# EUROPEAN PATENT SPECIFICATION

(11) **EP 3 154 995 B1**
(45) Date of publication and mention of the grant of the patent: **01.11.2023**
(21) Application number: 15806229.9
(22) Date of filing: 11.06.2015
(51) Int. Cl.: C07H 1/06, C07H 3/06, B01D 9/00

(54) **SEPARATION OF 2'-O-FUCOSYLLACTOSE FROM FERMENTATION BROTH**
TRENNUNG VON 2'-O-FUCOSYLLACTOSE AUS FERMENTATIONSBRÜHE
SÉPARATION DU 2'-O-FUCOSYLLACTOSE CONTENU DANS UN BOUILLON DE FERMENTATION

(30) Priority: 11.06.2014 DK 201470348
(43) Date of publication of application: 19.04.2017
(73) Proprietor: Glycom A/S, 2970 Hørsholm (DK)
(72) Inventor: MATWIEJUK, Martin, 22115 Hamburg (DE); CHASSAGNE, Pierre, F-63110 Beaumont (FR); OSZTROVSZKY, Györgyi, H-4600 Kisvárda (HU); HORVÁTH, Ferenc, H-2098 Pilisszentkereszt (HU); DEKANY, Gyula, Sinnamon Park, Queensland 4073 (AU)
(74) Representative: Inspicos P/S
(86) International application number: PCT/DK2015/050158
(87) International publication number: WO 2015/188834

(56) References cited:
- WO-A1-2011/150939
- WO-A1-2014/069625
- WO-A1-2014/086373
- WO-A2-2012/112777
- WO-A2-2014/009921
- DROUILLARD, S. ET AL.: 'Large-Scale Synthesis of H-Antigen Oligosaccharides by expressing Helicobacter pylori à1,2- Fucosyltransferase in Metabolically Engineered Escherichia coli Cells''.' ANGEWANDTE CHEMIE INTERNATIONAL EDITION. vol. 45, 2006, pages 1778 - 1780, XP055105681
- LEE, W.H. ET AL.: 'Whole Cell Biosynthesis of a Functional Oligosaccharide, 2'-fucosyllactose, using engineered Escherichia coli''.' MICROBIAL CELL FACTORIES. vol. 11, no. 48, 2012, XP021115889
- KUHN, R ET AL.: 'Fucosido-lactose, das Trisaccharid der Frauenmilch''.' CHEMISCHE BERICHTE. vol. 88, no. 8, 1955, pages 1135 - 1146, XP009074771
- KUHN, R ET AL.: 'Kristallisierte Fucosido-lactose''.' CHEMISCHE BERICHTE. vol. 89, no. 11, 1956, page 2513, XP055242299

## Description

### FIELD OF THE INVENTION

The invention relates to a method of crystallizing 2'-O-fucosyllactose (2'-FL) selectively from an aqueous media, particularly from biotechnological production of 2'-FL.

### BACKGROUND OF THE INVENTION

Many procedures are known for the biotechnological production of 2'-FL such as by fermentation with transformed *E. coli.* Some of them have been investigated only with qualitative analytical methods without mentioning the yield of 2'-FL or how to isolate it from the broth (WO 2010/070104, WO 2012/007481, Lee et al. Microb. Cell Fact. 11:48 (2012)). Preparative methods have been performed in lab scale and not exceeded the final titre of 25 g/l with regard to 2'-FL (Drouillard et al. Angew. Chem. Int. Ed. 45, 1778 (2006); M. Randriantsoa: Synthèse microbiologique des antigènes glucidiques des groupes sanguins, Thèse de Doctorat soutenue le 30/09/2008 à l' Université Joseph Fourier, Grenoble, France; WO 2012/097950, WO 2012/112777; Baumgärtner et al. Microb. Cell Fact. 12:40 (2013)). According to these publications, 2'-FL was then isolated from the aqueous culture medium or supernatant, in which the *E.coli* was fermented, by:
- separating the supernatant containing the product by centrifugation,
- adsorption of the product on a bed of activated charcoal that was washed with water to eliminate water-soluble contaminants like salts, amino acids and protein fragments, then the product was eluted with alcohol or aqueous alcohol, and then
- separation of 2'-FL from other carbohydrates like lactose and fucose by gel permeation chromatography or flesh chromatography on charcoal-celite bed.

The main drawback of this isolation method has been the need for chromatographic separation in order either to get the pure substance or to obtain at least a mixture that is enriched in the target compound but still contains undesired derivatives. Although repeated chromatographic separations can result in the improvement of the purity, its high cost and relatively long technological time to handle the feed solution and the column packing, to carry out the separation and optionally to regenerate the packing, especially in large or industrial scale, can be disadvantageous and/or cumbersome.

Crystallization or recrystallization is one of the simplest and cheapest methods to isolate a product from a reaction mixture, separate it from contaminations and obtain pure substance. Isolation or purification that uses crystallization makes the whole technological process robust and cost-effective, thus it is advantageous and attractive compared to other procedures. For this reason, 2'-FL, produced by synthetic chemical processes, has been isolated by crystallization (WO 2010/070616, WO 2011/150939, WO 2014/009921), or produced by fermentation (WO 98/12343, EP0870841) has been isolated by crystallization (WO 2014/ 069625).

However, because of the byproducts produced in the aqueous culture medium during fermentation of 2'-FL, there has been a continuing need for an efficient process for crystallizing 2'-FL from the aqueous culture medium.

### SUMMARY OF THE INVENTION

The invention relates to a method for selective crystallization of 2'-FL from an aqueous solution, derived from an aqueous culture medium comprising 2'-FL and a fucosylated carbohydrate other than 2'-FL by adding one or more C₁-C₄ alcohols to the solution, which aqueous solution is produced by a process comprising the steps of:
i. culturing, in an aqueous culture medium containing lactose, a genetically modified cell, preferably an E. coli cell, having a recombinant gene that encodes a 1,2-fucosyl transferase and able to produce 2'-FL by fucosylating lactose, and
ii. separating the aqueous solution from non-carbohydrate particulates and contaminants of the aqueous culture medium,
wherein the crystalline 2'-FL displays X-ray powder diffraction reflections, based on a measurement using CuKα radiation, at 16.98±0.20, 13.65±0.20 and 18.32±0.20 2Θ angles, more preferably at 16.98±0.20, 13.65±0.20, 18.32±0.20 and 21.70±0.20 2Θ angles, even more preferably at 16.98±0.20, 13.65±0.20, 18.32±0.20, 21.70±0.20 and 15.22±0.20 2Θ angles, most preferably at 16.98±0.20, 13.65±0.20, 18.32±0.20, 21.70±0.20, 15.22±0.20 and 20.63±0.20 2Θ angles, in particular at 16.98±0.20, 13.65±0.20, 18.32±0.20, 21.70±0.20, 15.22±0.20, 20.63±0.20 and 11.94±0.20 2Θ angles.

Also preferably, the aqueous solution is of 35-80 w/w% with regard to its carbohydrate content, 45-95 % of which is 2'-FL by weight and the 2'-FL/DFL ratio is not less than around 1:1.

### DETAILED DESCRIPTION OF THE INVENTION

As a result of recent developments in producing 2'-FL by culturing genetically modified *E. coli,* aqueous fermentation broths have been produced with a significantly higher titre of 2'-FL, e.g. at least 75 grams, more preferably at least 100 grams, particularly at around or more than 115 grams of 2'-FL per liter of the culture medium. However, fucosylated carbohydrates other than 2'-FL, particularly 2',3-di-O-fucosyllactose (DFL), as well as 2'-O-fucosyl-lactulose, have also been found as by-products in such high-titre aqueous fermentation broths in a DFL/2'-FL ratio of 2:8 to 1:9 by weight. Moreover, depending on the fermentation protocol, lactose could also be detected in such high-titre aqueous broths.

It has now been discovered that 2'-FL can be selectively crystallized from a high titred aqueous solution, obtained from such a high titred fermentation broth, by treating the aqueous solution with one or more C₁-C₄ alcohols, preferably methanol. This selective crystallization is particularly advantageous and surprising when the aqueous solution also contains other fucosylated carbohydrates and/or lactose. This selective crystallization provides 2'-FL of high purity in one step, and typically crystallization of batches of at least 100 g of 2'-FL, such as at least 1 kg, or at least 100 kg, or even at least 1 ton of 2'-FL, can be achieved with a wide range of concentrations of the contaminating sugar-like compounds in the aqueous fermentation broth.

Accordingly, one aspect of this invention is a method for selective crystallization of 2'-FL, characterized in that the crystallization is carried out from an aqueous solution, preferably a fermentation broth, comprising 2'-FL and a fucosylated carbohydrate other than 2'-FL by adding one or more C₁-C₄ alcohols to the aqueous solution. The resulting crystalline 2'-FL is a polymorph II as described in WO 2011/150939.

The term "C₁-C₄ alcohol" preferably refers to a mono- or dihydroxy alcohol having 1 to 4 carbon atoms such as methanol, ethanol, n-propanol, i-propanol, n-butanol, i-butanol, s-butanol, t-butanol, ethylene glycol, propylene glycol, etc. Preferably monohydroxy alcohol(s) is/are used such as methanol, ethanol, n-propanol, i-propanol, n-butanol, i-butanol, s-butanol or t-butanol.

In a preferred embodiment, the aqueous solution contains 35-80 w/w%, preferably 40-68 w/w%, more preferably 45-60 w/w%, particularly 50-56 w/w% of total carbohydrates including 2'-FL and other fucosylated and non-fucosylated carbohydrates. The above concentration ranges can be easily achieved by concentrating the aqueous supernatant from the fermentation, preferably after removing, e.g., cells, proteins, protein fragments, DNA, caramelized by-products, salts and/or charged molecules. These concentration ranges can be achieved in a conventional manner, e.g. by distilling water off at reduced pressure (20-100 mbars) and at ambient temperature up to 40-60 °C or by nanofiltration.

The resulting aqueous solution is then kept at around 40-60 °C, and one or more C₁-C₄ alcohols, preferably as hot, are added to the solution. The resulting aqueous alcoholic solution is stirred at the same temperature typically for 0.5-3 hours and allowed to cool to room temperature while spontaneous crystallization occurs. The crystallization can be initiated by adding seeding crystals.

Preferably, 45-95 % of the total carbohydrate content, including 2'-FL and other fucosylated and non-fucosylated carbohydrates, in the aqueous solution, before crystallization, is 2'-FL. The above defined crystallization conditions permit a wide range of 2'-FL content relative to the total carbohydrate content under which a selective crystallization of 2'-FL in high yield and good purity can be conducted. Accordingly the selective crystallization can be carried out from an aqueous solution that is of 35-80 w/w% with regard to its total carbohydrate content comprising 2'-FL and other fucosylated and non-fucosylated carbohydrates, and around 50 %, or around 60 %, or around 70 %, or around 80 %, or around 90 % of which is 2'-FL.

Preferably, the volume of the one or more C₁-C₄ alcohols to be added to the aqueous solution described above is about 3-9 fold relative to the weight of 2'-FL in that aqueous solution, more preferably about 4-8 volumes and even more preferably about 5-7 volumes relative to the weight of 2'-FL that can be added in one, two or even more portions.

Also preferably, the fucosylated carbohydrate other than 2'-FL in the aqueous solution is a fucosylated lactose other than 2'-FL and/or a fucosylated lactulose, preferably a fucosylated lactose other than 2'-FL. A fucosylated lactose other than 2'-FL can be any monofucosylated lactose other than 2'-FL that may be formed during fermentation as a result of a deficient, defective or impaired fucosylation other than an α-1,2-fucosylation on the galactose moiety of lactose (e.g. one leading to 3-O-fucosyllactose, 3-FL), or of fucose migration of 2'-FL under the cultivation condition or post-fermentive operations, or of fucose hydrolysis from multifucosylated, preferably difucosylated, lactose. A fucosylated lactose other than 2'-FL can also be a multifucosylated, preferably difucosylated, lactose that may be formed as a result of overfucosylation of lactose under the cultivation condition. The difucosylated lactose is preferably 2,2'-di-O-fucosyllactose or 2',3-di-O-fucosyllactose (DFL), particularly DFL as a characteristic by-product formed in a fermentative production of 2'-FL.

In a more preferred embodiment of a method for the selective crystallization of 2'-FL from an aqueous solution comprising 2'-FL and DFL, the 2'-FL/DFL ratio by weight can vary in wide ranges without the peril that DFL significantly co-crystallizes beside 2'-FL, but is not less than 1:1. Thus the selective crystallization effectively works at a 2'-FL/DFL ratio more than 1:1, preferably more than 2:1, more preferably more than 4:1, even more preferably more than 6:1, particularly around between 8:1 and 12:1.

The aqueous solution containing 2'-FL and DFL from which 2'-FL can be selectively crystallized may comprise further carbohydrate-like contaminants like 2-O-fucosyl lactulose, lactose, lactulose, fucose, glucose, galactose and the like. These contaminants might have been formed during fermentation or in the post-fermentive purification/isolation steps e.g. by rearrangement (e.g. 2-O-fucosyl lactulose, lactulose) or by hydrolysis (e.g. fucose, glucose, galactose, lactose), or can be an unconsumed educt or ingredient added during the fermentation (e.g. lactose as acceptor, glucose as carbon source). These sugar-like contaminants are detected in the aqueous solution to be used for crystallization in a concentration not more than 1-2 w/w% (individually), or 5-7 w/w% (altogether). Typically, the aqueous solution containing 2'-FL and DFL, and intended to use for crystallization purpose may further contain lactose as unused acceptor added during the fermentation for making 2'-FL from lactose by fucosylation in less than 1 w/w%.

Yet preferably, the method for the crystallization of 2'-FL from an aqueous solution comprising 2'-FL and a fucosylated carbohydrate other than 2'-FL, preferably DFL, is conducted by adding only one C₁-C₄ alcohol, more preferably a C₁-C₂ alcohol (that is methanol or ethanol), among which methanol is the most preferable choice for an alcohol.

According to a preferred embodiment of the method for selectively crystallizing 2'-FL, the aqueous solution used for crystallization comprises 2'-FL, DFL and optionally lactose in a concentration of 40-68 w/w%, more preferably 45-60 w/w%, particularly 50-56 w/w%. The proportion of 2'-FL in the above defined carbohydrate mass of the above defined aqueous solution is more than around 80 %, more preferably more than about 85% and particularly about 90 %. The 2'-FL/DFL ratio in the above defined carbohydrate mass is around 8:1 to 12:1 by weight. To this aqueous solution, preferably after warming it up to around 45-55 °C and while being stirred methanol is added, preferably in 2-3 approximately equal portions, while the temperature is maintained at the given range. After addition of the whole amount of methanol, which is typically 4-8 volumes, more preferably 5-7 volumes relative to the weight of 2'-FL in the aqueous solution, the temperature of the stirred mixture is kept at the same level than that was at methanol addition for 0.5-2 hours, then the mixture is allowed to cool down to room temperature at which the stirring is continued for 12-24 hours. The crystallization spontaneously occurs during the addition of methanol or at the cooling phase, but the crystallization can be facilitated by addition of 2'-FL seeding crystals.

According to another preferred realization of the method for selective crystallization of 2'-FL the starting aqueous mixture comprises 2'-FL, DFL and optionally lactose in a concentration of 50-80 w/w%, more preferably 55-70 w/w%, particularly around 60-68 w/w%. The proportion of 2'-FL in the above defined carbohydrate mass of the above defined aqueous solution is around 48-65 %, and 2'-FL/DFL ratio in the above defined carbohydrate mass is around 1:1 to 2:1 by weight. To this aqueous solution, preferably after warming it up to around 55-60 °C and while being stirred, methanol is added, preferably in 2-3 approximately equal portions, while the temperature is maintained at the given range. After addition of the whole amount of methanol, which is typically around 5-8.5 volumes, more preferably around 6.5-7.5 volumes relative to the weight of 2'-FL in the aqueous solution, the temperature of the stirred mixture is kept at the same temperature than that was at methanol addition for 0.5-2 hours, then the mixture is allowed to cool down to room temperature at which the stirring is continued for 12 hours to 3 days. The crystallization spontaneously occurs during the addition of methanol or at the cooling phase, but the crystallization can be facilitated by addition of 2'-FL seeding crystals.

The aqueous solution comprising 2'-FL and a fucosylated carbohydrate other than 2'-FL, preferably DFL, for selectively crystallizing 2'-FL according to the present invention described above in details is produced by a process comprising the steps:
i. culturing, in an aqueous culture medium containing lactose, a genetically modified cell, preferably an *E. coli* cell, having a recombinant gene that encodes an α-1,2-fucosyl transferase and able to produce 2'-FL by fucosylating lactose, and
ii. separation of the aqueous carbohydrate fraction comprising 2'-FL and a fucosylated lactose other than 2'-FL, preferably DFL, from non-carbohydrate particulates and contaminants of the fermentation broth.

Step ii) in the above process may comprise a conventional demineralization step during which minerals, salts and other charged molecules are extracted from the aqueous solution before crystallization. The demineralization can be conducted by using conventional ion exchange resins, namely passing the aqueous solution through a cation exchange resin in H⁺-form and an anion exchange resin in free base form. The cation exchange resin is preferably a strong exchanger, and the anion exchange resin is a weak exchanger. The ion exchange resins, beside removing salts and charged molecules from the solution, can physically adsorb proteins, DNA and colorizing/caramel bodies that optionally left in the solution after previous purification steps. Alternatively, the demineralization can be conducted by means of a conventional electrodialysis. The solution obtained by any above ways can then be concentrated by either a conventional evaporation step or a conventional nanofiltration step.

In addition, step ii) in the above process can further comprise a conventional charcoal treatment, preferably before the demineralization step, to remove colour bodies and optionally water soluble biojunk optionally left from previous purification steps. The carbohydrate compounds have strong affinity to be adsorbed on charcoal in aqueous medium thus water-soluble contaminants can be easily washed away with (distilled) water. The carbohydrates can then be eluted from the charcoal bed with alcohol or aqueous alcohol.

Moreover, step ii) in the above process can comprise a conventional clarification step for removing cells, cells fragments and proteins after fermentation, preferably prior to the charcoal treatment described above. The clarification can be done in a conventional manner, e.g. by sedimentation in centrifuge producing a clarified or partially clarified supernatant solution. Alternatively, the fermentation broth can be subjected to ultrafiltration in a conventional manner to remove high molecular weight components. The semipermeable membrane used for ultrafiltrating a 2'-FL fermentation broth can suitably have a cut off of 5-50 kDa, preferably 10-25 kDa, more preferably around 15 kDa. Depending on the characteristics of the fermentation broth to be clarified combination of higher and lower cut off membranes (in this order) within the above given range may be employed. Optionally, centrifugation or ultrafiltration can be followed by nanofiltration, during which the aqueous solution containing 2'-FL and accompanying carbohydrates is concentrated in a conventional manner before it is treated with charcoal. In this nanofiltration step, its membrane can have a pore size that ensures retention of 2'-FL having a molecular weight of 488; so typically a 200-300 Da cut off membrane can be used.

Step i) in the above process preferably comprises the production of 2'-FL in high titre. An *E. coli* strain, particularly an *E. coli* LacZ⁻Y⁺ strain, is used having only one recombinant glycosyl transferase which is an α-1,2-fucosyl transferase, preferably an α-1,2-fucosyl transferase encoded by the futC gene from *Helicobacter pylori.* For making a mixture of 2'-FL in high yield, the method preferably comprises: a) providing, to the culture medium, a carbon and energy source and at least 50, preferably at least 75, more preferably at least 100, even more preferably at least 125 grams of lactose based on 1 liter of initial culture volume. The method also preferably comprises: b) providing lactose to the culture medium for more than 4 days, preferably up to 7 days, preferably in a continuous manner. It is particularly preferred that the method comprises both steps a) and b). It is also particularly preferred that the final volume of the culture medium is not more than three-fold, more preferably not more than two-fold, particularly less than two-fold of the volume of the culture medium before providing the lactose and the carbon and energy source, preferably glycerol to the culture medium. The culturing is preferably is performed in the following way:
- a first phase of exponential cell growth ensured by a carbon-based substrate, and
- a second phase of cell growth limited by a carbon and energy source which is added continuously, together with the lactose that is added continuously.

The *E. coli* is preferably cultured continuously, preferably for at least 4 days, particularly up to 7 days, but not more than about 9-10 days, preferably at a temperature of 30 to 35 °C, and preferably with continuous agitation, continuous aeration and continuous feeding of the carbon and energy source and lactose. The so-produced fermentation broth preferably comprises at least 75 grams, more preferably at least 100 grams, particularly up to 115 grams per liter of the culture medium in the supernatant. In addition, extracellular fraction contains DFL too in around 2.5-20 w/w% relative to 2'-FL. The 2'-FL + DFL mixture optionally contains fucosylated lactulose ((2'-O-fucosyl-lactulose), that is produced in the culture medium, and/or lactose as unconsumed acceptor.

### EXAMPLES

### Example 1

### Producing 2'-FL in high yield

### Bacterial strains and inoculum preparation:

Engineered *E. coli* was constructed from *E. coli* K strain in accordance with WO 01/04341 and Drouillard et al. Angew. Chem. Int. Ed. Eng. 45, 1778 (2006), by deleting genes that are liable to degrade lactose, the oligosaccharide products and their metabolic intermediates, inter alia the lacZ, lacA and wcaJ genes, maintaining manB, manC, gmd and wcaG genes involved in the GDP-fucose biosynthesis, and inserting *H. pylori* futC gene for α-1,2-fucosyl transferase, as only glycosyl transferase.

### Fermentation condition:

Glucose, glycerol, isopropyl thio-β-D-galactopyranoside (IPTG) and lactose were each sterilized at 120 °C.

The culture was carried out in a 3 I fermenter containing 1.5 I of mineral culture medium (Samain et al. J. Biotechnol. 72, 33 (1999)). The temperature was kept at 33 °C and the pH regulated at 6.8 with 28% NH₄OH. The inoculum (1 % of the volume of the basal medium) consisted in a LB medium and the culture of the producing strain. The exponential growth phase started with the inoculation and stopped until exhaustion of the carbon source (glucose 17.5 g/l) initially added to the medium. The inducer (isopropyl thio-β-D-galactopyranoside, IPTG, 1-2 ml of a 50 mg/ml solution) was added at the end of the exponential phase. Then a fed-batch was realized, using 1 I of a feed solution containing 500 g of glycerol and 160-200 g of lactose dissolved in water, which was added to the culture during 4-7 days. At the end of the fermentation, the 2'-FL concentration varied between 68-114 g/l, and the 2'-FL:DFL ratio varied between around 80:20 to 88:12 in the mixture produced. Fucosylated lactulose (2'-O-fucosyl-lactulose) was no more than 1 % in this mixture.

### Example 2

### Purification of the broth

Cells and proteins were removed by ultrafiltration and the obtained solution was concentrated by nanofiltration to around 400 ml (the solution contained ≈93 g of 2'-FL, ≈10.5 g of DFL and ≈0.5 g of lactose). The solution was then treated with charcoal (8 g) to decolorize. The decolorized solution was eluted through a strong cation exchange resin (H⁺ form) and a weak anion exchange resin (free base form) to demineralize it and catch the remaining colour (eluent: demineralized water). No separation of carbohydrates was observed. The carbohydrate positive fractions were pooled and concentrated to 216 g at reduced pressure (20-30 mbars) at 40 °C. The analysis of the resulting almost colourless solution showed no loss of 2'-FL, DFL and lactose by adsorption on the charcoal and the resins, and the presence of ≈10-15 g of non-analysed other contaminants.

### Example 3

### Selective crystallization of 2'-FL from a 2'-FL/DFL ≈8.9:1 mixture

The concentrate from Example 2 was then heated to 50 °C and 280 ml of methanol was added slowly under stirring while maintaining the temperature. After addition of seeding crystals (50 mg), another portion of methanol (280 ml) was added slowly at 50 °C and the stirring was continued at this temperature for 1 hour. The crystallizing solution was allowed to cool to room temperature and stirred for 18 hours. The solid was filtered, washed with cold methanol and dried under vacuum (15 mbar) at 50 °C for 16 hours to give 82 g of white crystals. 2'-FL assay by HPLC: 97.8 %, by IC: 97.1 %; water content: 0.64 % (KF); rel. purity of 2'-FL by HPLC: 99.1 %, by IC: 99.4 %; yield of the selective crystallization: 86 %.

### Example 4

### Selective crystallization of 2'-FL from a 2'-FL/DFL ≈1:1 mixture

The mother liquor from Example 3 was freeze-dried. A sample from the freeze-dried powder (20 g, containing ≈8.4 g of 2'-FL, ≈8.4 g of DFL and ≈0.3 g of lactose) was taken up in water (5 ml) and methanol (60 ml) at 60 °C, the mixture was seeded and stirred for 1 hour then cooled down to room temperature and stirred for 3 days. The white crystals were filtered off and dried to give 6.9 g. 2'-FL assay by HPLC: 93.3 %.

### Example 5

### Selective crystallization of 2'-FL from a 2'-FL/DFL ≈1.7:1 mixture

The crystallization was performed according to Example 3 from a solution containing ≈23 g of 2'-FL, ≈2.5 g of DFL and ≈0.15 g of lactose (100 ml) obtained from a fermentation broth after ultra- and nanofiltration to give 18.7 g of 2'-FL. Methanol was distilled off from the mother liquor and concentrated to weight 11 g (containing ≈4.2 g of 2'-FL and ≈2.5 g of DFL). The resulting syrup was warmed up to 60 °C and methanol was added (30 ml) in portions, between two portions of methanol the solution was seeded. After 1 hour of stirring at reflux the suspension was allowed to cool to room temperature, the crystals were filtered off, washed and dried to provide 3.2 g of 2'-FL.

### Example 6

### Selective crystallization of 2'-FL from a 2'-FL/DFL ≈10.2:1 mixture

An aqueous solution containing 173 g of 2'-FL, 17 g of DFL and 4 g of lactose (600 ml), obtained from a fermentation broth after ultrafiltration, nanofiltration and treatment with charcoal and ion exchange resins as disclosed in Example 2 was concentrated by vacuum distillation (70-100 mbar, 50-60 °C) to around 270 ml. To the resulting mixture methanol (1040 ml) was added while keeping the solution at around 50 °C. Seeding crystals were then added and the stirring was continued at 50 °C for 1 hour and at room temperature for 15 hours. The crystals were filtered off, washed with cold methanol and dried under vacuum to give 158 g of white solid (91 %).

## Claims

1. Method for crystallization of 2'-FL, **characterized in that** the crystallization is carried out from an aqueous solution comprising 2'-FL and a fucosylated carbohydrate other than 2'-FL by adding one or more C₁-C₄ alcohols to the solution, which aqueous solution is produced by a process comprising the steps of:
i. culturing, in an aqueous culture medium containing lactose, a genetically modified cell, preferably an E. coli cell, having a recombinant gene that encodes a 1,2-fucosyl transferase and able to produce 2'-FL by fucosylating lactose, and
ii. separating the aqueous solution from non-carbohydrate particulates and contaminants of the aqueous culture medium,
wherein the crystalline 2'-FL displays X-ray powder diffraction reflections, based on a measurement using CuKα radiation, at 16.98±0.20, 13.65±0.20 and 18.32±0.20 2Θ angles, more preferably at 16.98±0.20, 13.65±0.20, 18.32±0.20 and 21.70±0.20 2Θ angles, even more preferably at 16.98±0.20, 13.65±0.20, 18.32±0.20, 21.70±0.20 and 15.22±0.20 2Θ angles, most preferably at 16.98±0.20, 13.65±0.20, 18.32±0.20, 21.70±0.20, 15.22±0.20 and 20.63±0.20 2Θ angles, in particular at 16.98±0.20, 13.65±0.20, 18.32±0.20, 21.70±0.20, 15.22±0.20, 20.63±0.20 and 11.94±0.20 2Θ angles.

2. The method according to claim 1, wherein the aqueous solution is of 35-80 w/w% with regard to its carbohydrate content.

3. The method according to any of the claims 1 or 2, wherein 45-95 % of the carbohydrates in the aqueous solution is 2'-FL by weight.

4. The method according to any of the claims 1 to 3, wherein 3-9 volumes of alcohol are added relative to the weight of 2'-FL.

5. The method according to any of the claims 1 to 4, wherein the fucosylated lactose other than 2'-FL is DFL.

6. The method according to claim 5, wherein the 2'-FL/DFL ratio is not less than around 1:1 by weight.

7. The method according to claim 6, wherein the 2'-FL/DFL ratio is more than 1:1, preferably more than 2:1, more preferably more than 4:1, even more preferably more than 6:1, particularly between 8:1 and 12:1

8. The method according to claim 6 or 7, wherein the aqueous solution is of 40-68 w/w%, more preferably 45-60 w/w%, particularly 50-56 w/w%, and the 2'-FL/DFL ratio is around 8:1 to 12:1 by weight.

9. The method according to claim 6 or 7, wherein the aqueous solution is of 50-80 w/w%, more preferably 55-70 w/w%, particularly around 60-68 w/w% and the 2'-FUDFL ratio is around 1:1 to 2:1 by weight.

10. The method according to any of the precedent claims, wherein the aqueous solution further contains lactose.

11. The method according to any of the precedent claims, wherein the C₁-C₄ alcohol is methanol or ethanol, preferably methanol.

12. The method according to any of the precedent claims, wherein step ii. further comprises an ion exchange resin treatment to demineralize and/or decolorize the fermentation broth obtained in step i.

13. The method according to claim 12, wherein the ion exchange resin treatment is preceded by ultrafiltration of the fermentation broth obtained in step i.

14. The method according to any one of the preceding claims, wherein the genetically modified cell is an *E. coli* LacZ⁻Y⁺ cell having only one recombinant glycosyl transferase which is an α-1,2-fucosyl transferase.

## Patentansprüche

1. Ein Verfahren zur Kristallisation von 2'-FL, **dadurch gekennzeichnet, dass** die Kristallisation ausgehend von einer wässrigen Lösung durchgeführt wird, die 2'-FL und ein fucosyliertes Kohlenhydrat umfasst, welches sich von 2'-FL unterscheidet, wobei der Lösung zu diesem Zwecke ein oder mehrere C₁-C₄-Alkohole zugesetzt werden, wobei die wässrige Lösung mittels eines Prozesses hergestellt wird, welches die folgenden Schritte umfasst:
i. Kultivieren, in einem wässrigen Kulturmedium, das Lactose enthält, einer gentechnisch veränderten Zelle, vorzugsweise einer E.-coli-Zelle, die ein rekombinantes Gen aufweist, welches für 1,2-Fucosyltransferase codiert, wobei sie dazu befähigt ist, 2'-FL durch Fucosylieren von Lactose zu produzieren, und
ii. Abtrennen der wässrigen Lösung von den nichtkohlenhydratartigen Stoffen und Verunreinigungen des wässrigen Kulturmediums,
wobei das kristalline 2'-FL Pulverröntgenbeugungssignale, auf Basis einer Messung unter Verwendung von CuKα-Strahlung, bei 2θ Winkeln von 16,98±0,20, 13,65±0,20 und 18,3210,20, insbesondere bei 2θ Winkeln von 16,98±0,20, 13,65±0,20, 18,32±0,20 und 21,70±0,20, noch stärker bevorzugt bei 2θ Winkeln von 16,98±0,20, 13,65±0,20, 18,32±0,20, 21,70±0,20 und 15,22±0,20, mit dem größten Vorzug bei 2θ Winkeln von 16,98±0,20, 13,65±0,20, 18,32±0,20, 21,70±0,20, 15,22±0,20 und 20,63±0,20, insbesondere bei 2θ Winkeln von 16,98±0,20, 13,65±0,20, 18,32±0,20, 21,70±0,20, 15,22±0,20, 20,63±0,20 und 11,94±0,20 zeigt.

2. Das Verfahren gemäß Anspruch 1, wobei die wässrige Lösung bezüglich ihres Kohlenhydratgehalt 35 bis 80 Gew-%ig ist.

3. Das Verfahren gemäß einem beliebigen der Ansprüche 1 oder 2, wobei 2'-FL nach Gewicht 45 bis 95 % der Kohlenhydrate in der wässrigen Lösung ausmacht.

4. Das Verfahren gemäß beliebigen der Ansprüche 1 bis 3, wobei unter Bezugnahme auf das Gewicht an 2'-FL 3 bis 9 Volumina an Alkohol zugesetzt werden.

5. Das Verfahren gemäß einem beliebigen der Ansprüche 1 bis 4, wobei es sich bei der fucosylierten Lactose, die nicht 2'-FL entspricht, um DFL handelt.

6. Das Verfahren gemäß Anspruch 5, wobei das 2'-FL/DFL-Verhältnis nicht niedriger als ungefähr 1:1 nach Gewicht ist.

7. Das Verfahren gemäß Anspruch 6, wobei das 2'-FL/DFL-Verhältnis höher als 1:1, vorzugsweise höher als 2:1, stärker bevorzugt höher als 4:1, noch stärker bevorzugt höher als 6:1 ist, wobei es insbesondere zwischen 8:1 und 12:1 liegt.

8. Das Verfahren gemäß Anspruch 6 oder 7, wobei die wässrige Lösung 40 bis 68 Gew-%ig , stärker bevorzugt 45 bis 60 Gew-%ig , insbesondere 50 bis 56 Gew-%ig ist und das 2'-FL/DFL-Verhältnis ungefähr 8:1 bis 12:1 nach Gewicht beträgt.

9. Das Verfahren gemäß Anspruch 6 oder 7, wobei die wässrige Lösung 50 bis 80 Gew-%ig , stärker bevorzugt 55 bis 70 Gew-%ig , insbesondere ungefähr 60 bis 68 Gew-%ig) ist und das 2'-FL/DFL-Verhältnis ungefähr 1:1 bis 2:1 nach Gewicht beträgt.

10. Das Verfahren gemäß einem beliebigen der vorhergehenden Ansprüche, wobei die wässrige Lösung weiterhin Lactose enthält.

11. Das Verfahren gemäß einem beliebigen der vorhergehenden Ansprüche, wobei es sich bei dem C₁-C₄-Alkohol um Methanol oder Ethanol, vorzugsweise Methanol, handelt.

12. Das Verfahren gemäß einem beliebigen der vorhergehenden Ansprüche, wobei der Schritt ii. weiterhin eine Behandlung mittels Ionenaustauscherharz umfasst, um das Fermentationsmedium, welches im Schritt i. erhalten wurde, zu entmineralisieren und/oder zu entfärben.

13. Das Verfahren gemäß Anspruch 12, wobei im Vorfeld der Behandlung mittels Ionenaustauscherharz eine Ultrafiltration des Fermentationsmediums, wie es im Schritt i. erhalten wurde, erfolgt.

14. Das Verfahren gemäß einem beliebigen der vorhergehenden Ansprüche, wobei es sich bei der gentechnisch veränderten Zelle um eine *E. coli* LacZ⁻Y⁺-Zelle handelt, die über lediglich eine rekombinante Glycosyltransferase verfügt, bei welcher es sich um eine α-1,2-Fucosyltransferase handelt.

## Revendications

1. Un procédé de cristallisation du 2'-FL, **caractérisé en ce que** la cristallisation est réalisée à partir d'une solution aqueuse comprenant du 2'-FL et un sucre fucosylé différent du 2'-FL, par addition d'un ou de plusieurs alcools en C₁-C₄ à la solution, laquelle solution aqueuse est produite par un procédé comprenant les étapes consistant à :
i. cultiver, dans un milieu de culture aqueux contenant du lactose, une cellule génétiquement modifiée, de préférence une cellule d'E. coli, ayant un gène recombinant qui code pour une 1,2-fucosyl transférase et capable de produire du 2'-FL par fucosylation du lactose et,
ii. séparer la solution aqueuse des particules non glucidiques et des contaminants du milieu de culture aqueux,
où le 2'-FL cristallin présente des réflexions de diffraction des rayons X sur poudre, sur la base d'une mesure utilisant le rayonnement CuKα, aux angles 16,98 ± 0,20, 13,65 ± 0,20 et 18,32 ± 0,20 2Θ, plus préférablement aux angles 16,98 ± 0,20, 13,65 ± 0,20, 18,32 ± 0,20 et 21,70 ± 0,20 2Θ, encore plus préférablement aux angles 16,98 ± 0,20, 13,65 ± 0,20, 18,32 ± 0,20, 21,70 ± 0,20 et 15,22 ± 0,20 2Θ, de manière préférée entre toutes aux angles 16,98 ± 0,20, 13,65 ± 0,20, 18,32 ± 0,20, 21,70 ± 0,20,, 15,22 ± 0,20 et 20,63 ± 0,20 2Θ, en particulier aux angles 16,98 ± 0,20, 13,65 ± 0,20, 18,32 ± 0,20, 21,70 ± 0,20, 15,22 ± 0,20, 20,63 ± 0,20 et 11,94 ± 0,20 2Θ.

2. Le procédé selon la revendication 1, dans lequel la solution aqueuse est de 35 à 80 % m/m par rapport à sa teneur en glucides.

3. Le procédé selon l'une quelconque des revendications 1 ou 2, dans lequel 45 à 95 % des glucides de la solution aqueuse est du 2'-FL, en masse.

4. Le procédé selon l'une quelconque des revendications 1 à 3, dans lequel 3 à 9 volumes d'alcool sont ajoutés par rapport à la masse de 2'-FL.

5. Le procédé selon l'une quelconque des revendications 1 à 4, dans lequel le lactose fucosylé différent du 2'-FL est le DFL.

6. Le procédé selon la revendication 5, dans lequel le rapport 2'-FL/DFL n'est pas moins d'environ 1 : 1 en masse.

7. Le procédé selon la revendication 6, dans lequel le rapport 2'-FL/DFL est de plus de 1 : 1, préférablement de plus de 2 : 1, plus préférablement de plus de 4 : 1, encore plus préférablement de plus de 6 : 1, en particulier compris entre 8 : 1 et 12 : 1.

8. Le procédé selon la revendication 6 ou 7, dans lequel la solution aqueuse est de 40 à 68 % m/m, plus préférablement de 45 à 60 % m/m, en particulier de 50 à 56 % m/m et le rapport 2'FL/DFL est d'environ 8 : 1 à 12 : 1 en masse.

9. Le procédé selon la revendication 6 ou 7, dans lequel la solution aqueuse est de 50 à 80 % m/m, plus préférablement de 55 à 70 % m/, en particulier d'environ 60 à 68 % m/m et le rapport 2'-FL/DFL est d'environ 1 : 1 à 2 : 1 en masse.

10. Le procédé selon l'une quelconque des revendications précédentes, dans lequel la solution aqueuse contient en outre du lactose.

11. Le procédé selon l'une quelconque des revendications précédentes, dans lequel l'alcool en C₁-C₄ est le méthanol ou l'éthanol, de préférence le méthanol.

12. Le procédé selon l'une quelconque des revendications précédentes, dans lequel l'étape ii. comprend en outre un traitement par résine échangeuse d'ions pour déminéraliser et/ou décolorer le bouillon de fermentation obtenu dans l'étape i.

13. Le procédé selon la revendication 12, dans lequel le traitement par résine échangeuse d'ions est précédé par une ultrafiltration du bouillon de fermentation obtenu dans l'étape i.

14. Le procédé selon l'une quelconque des revendications précédentes, dans lequel la cellule génétiquement modifiée est une cellule d'*E*. *coli* LacZ⁻Y⁺ ayant seulement une glycosyl transférase recombinante qui est une α-1,2-fucosyl transférase.
